(19) Europäisches Patentamt
European Patent Office
Office européen des brevets

(11) **EP 4 566 533 A1**

(12) **EUROPEAN PATENT APPLICATION**

(43) Date of publication:
**11.06.2025 Bulletin 2025/24**

(21) Application number: **24215980.4**

(22) Date of filing: **28.11.2024**

(51) International Patent Classification (IPC):
**A61B 5/113** *(2006.01)*   **A61B 5/287** *(2021.01)*
**A61B 5/367** *(2021.01)*   **A61B 5/00** *(2006.01)*

(52) Cooperative Patent Classification (CPC):
**A61B 5/287; A61B 5/113; A61B 5/1135;**
**A61B 5/367; A61B 5/6852**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.11.2023 US 202318523526**

(71) Applicant: **Biosense Webster (Israel) Ltd.**
**2066717 Yokneam (IL)**

(72) Inventors:
• **SIGAL, Alona**
**2066717 YOKNEAM (IL)**
• **TURGEMAN, Aharon**
**2066717 YOKNEAM (IL)**
• **BAR-TAL, Meir**
**2066717 YOKNEAM (IL)**
• **NOVOGRODSKY, Ben Ami**
**2066717 YOKNEAM (IL)**
• **SILBERSCHEIN, Erez**
**2066717 YOKNEAM (IL)**

(74) Representative: **Carpmaels & Ransford LLP**
**One Southampton Row**
**London WC1B 5HA (GB)**

(54) **RESPIRATION COMPENSATION FOR MAPPING**

(57) A system and method are disclosed. The system and method include obtaining respiration data of a patient via at least one sensor, obtaining probe location data for a probe positioned within a cavity, generating probe-cavity location data by compensating the respiration data from the probe location data, identifying periods when the probe is stable relative to a cavity boundary based on the probe-cavity location data, and capturing data based on generated probe-cavity location during identified periods to produce mapping.

FIG. 1

EP 4 566 533 A1

**Description**

FIELD OF INVENTION

The present application provides systems, apparatuses, and methods for compensating for respiration in mapping.

BACKGROUND

Surgical procedures involving cavities or chambers, such as the heart, require accurate information regarding a position of the probe or catheter in the cavity. In one aspect, mapping of a heart chamber is critical in order to identify issues regarding cardiac arrhythmia (e.g., atrial fibrillation (AF)), which can be treated via intra-body procedures.

Respiration is divided into breathing in (inhalation or inspiration) and breathing out (exhalation or expiration). Due to human physiology, the diaphragm moves up and down during this process and displaces the heart in a cyclical manner. During the expiration phase, there is a moment called the end-expirium phase in which this displacement from the diaphragm is minimal for a period of time.

SUMMARY

A system and method are disclosed. The system and method include obtaining respiration data of a patient via at least one sensor, obtaining probe location data for a probe positioned within a cavity, generating probe-cavity location data by compensating the respiration data from the probe location data, identifying periods when the probe is stable relative to a cavity boundary based on the probe-cavity location data, and capturing data based on generated probe-cavity location during identified periods to produce mapping.

BRIEF DESCRIPTION OF THE DRAWINGS

A more detailed understanding can be had from the following description, given by way of example in conjunction with the accompanying drawings wherein:

FIG. 1 depicts an example catheter-based electrophysiology mapping and ablation system according to one or more embodiments;
FIG. 2 is an example image produced using the disclosed embodiments;
FIG. 3 is a schematic diagram illustrating a system according to one aspect;
FIG. 4A is a first portion of a flow diagram showing aspects of the disclosed embodiments;
FIG. 4B is a second portion of the flow diagram from FIG. 4A;
FIG. 5 is a graph illustrating respiration indicator signals during ablation;
FIG. 6 is a graph illustrating an exemplary low pass and derivative filter with respect to weights;
FIG. 7A is a first portion of a flow chart for a pre-processing phase;
FIG. 7B is a second portion of the flow chart from FIG. 7A;
FIG. 8 is a graph illustrating respiration vectors during ablation;
FIG. 9 is a graph illustrating a recency factor;
FIG. 10 is a graph illustrating a speed factor;
FIG. 11 is a graph illustrating an ablation factor;
FIG. 12 is a graph illustrating multiple stability factors;
FIG. 13 illustrates a flow diagram for defining a split curve;
FIG. 14 illustrates a flow diagram of a method according to one embodiment;
FIG. 15 illustrates a method of capturing mapping data with respiration compensation; and
FIG. 16 illustrates a plot of respiration data points with and without respiration compensation.

DETAILED DESCRIPTION

As disclosed herein, systems, apparatuses and methods are provided that determine or estimate motion of a probe while inside of a chamber by accounting for respiration motion. The term probe is used interchangeably with the term catheter herein, and one skilled in the art would understand that any type of location sensing device could be implemented with the configurations disclosed herein.

A system and method are disclosed. The system and method include obtaining respiration data of a patient via at least one sensor, obtaining probe location data for a probe positioned within a cavity, generating probe-cavity location data by compensating the respiration data from the probe location data, identifying periods when the probe is stable relative to a cavity boundary based on the probe-cavity location data, and capturing data based on generated probe-cavity location during identified periods to produce mapping.

The method includes obtaining respiration data of a patient via at least one sensor, obtaining probe location data for a probe positioned within a cavity, generating compensated location data by compensating the obtained probe location data with the obtained respiration data, and producing a mapping based on the generated compensated location data. The method may include applying a filter to the respiration data. The obtaining respiration data may include obtaining respiration indicators (abbreviated herein as "RI") via the at least one sensor. The obtaining respiration data may include converting the respiration indicators by using singular value decomposition (SVD) to obtain a first Eigenvector and a first Eigenvector derivative, the first Eigenvector corresponding to a primary respiration signal and the first Eigenvector derivative corresponding to a phase shifted respiration signal. The primary respiration signal and the phase shifted respiration signal are transferred from two dimensions into a three-dimensional ellipsoid based on a correlation matrix. The correlation matrix is determined based on weighted factors including at least one of: age of the respiration data; speed of the probe; and depth of respiration. The method may include generating an image including estimated respiratory motion based on the respiration data, probe motion based on the probe location data, and probe-cavity motion based on the probe-cavity location data. The method may include notifying a surgeon of the periods when the probe is stable relative to the cavity boundary. The notifying a surgeon includes notifying the surgeon of the periods when the probe is stable relative to the cavity boundary via visual indicators displayed on a monitor. The method may include identifying site stability sites based on the periods when probe speed is less than 2 mm/second for at least three seconds.

The system may include a probe configured to be inserted into an intra-body cavity of a patient, at least one sensor configured to obtain respiration data, the probe and the at least one sensor being configured to obtain probe location data, and a processor configured to generate compensated location data by compensating the obtained probe location data with the obtained respiration data, and produce a mapping based on the generated compensated location data. The processor may be configured to apply a filter to the respiration data and the probe location data. The processor may be configured to generate respiration indicators based on the respiration data from the at least one sensor. The processor may be configured to convert the respiration indicators by using singular value decomposition (SVD) to obtain a first Eigenvector and a first Eigenvector derivative, the first Eigenvector corresponding to a primary respiration signal and the first Eigenvector derivative corresponding to a phase shifted respiration signal. The primary respiration signal and the phase shifted respiration signal are transferred from two dimensions into a three-dimensional ellipsoid based on a correlation matrix. The correlation matrix is determined based on weighted factors including at least one of: age of the respiration data; speed of the probe; and depth of respiration. The processor may be configured to generate an image including estimated respiratory motion based on the respiration data, probe motion based on the probe location data, and probe-cavity motion based on the probe-cavity location data. The processor may be configured to notify a surgeon of the periods when the probe is stable relative to the cavity boundary. The notifying the surgeon of the periods when the probe is stable relative to the cavity boundary includes displaying visual indicators on a monitor. The processor may be configured to identify site stability sites based on the periods when probe speed is less than 2 mm/second for at least three seconds.

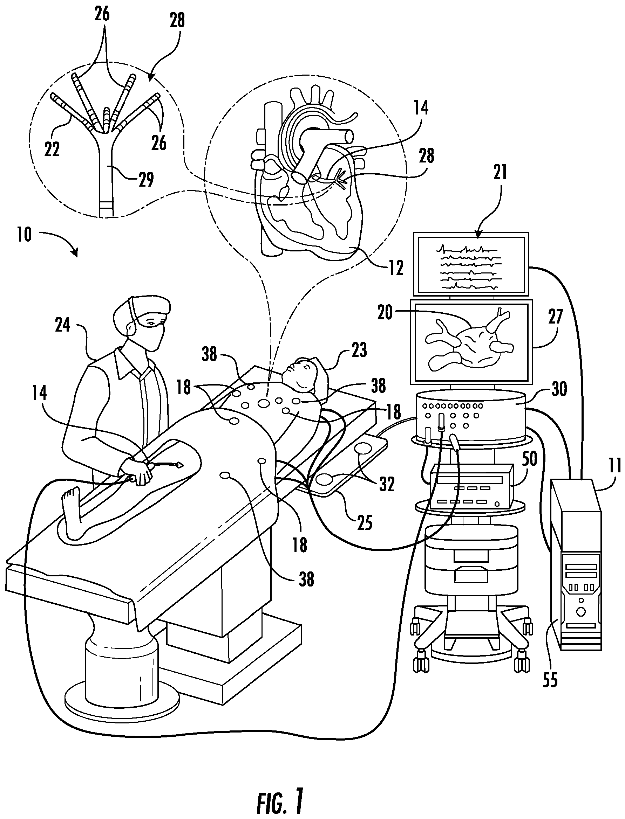

Reference is made to FIG. 1 showing an example system (e.g., medical device equipment and/or catheter-based electrophysiology mapping and ablation system), shown as system 10, in which one or more features of the subject matter herein can be implemented according to one or more embodiments. All or part of the system 100 can be used to collect information (e.g., biometric data and/or a training dataset) and/or used to implement a machine learning and/or an artificial intelligence algorithm as described herein. The system 10, as illustrated, includes a recorder 11, a heart 12, a catheter 14, a model or anatomical map 20, an electrogram 21, a spline 22, a patient 23, a physician 24 (which is representative of any medical professional, technician, or clinician), a location pad 25, one or more electrodes 26, a display device 27, a distal tip 28, a sensor 29, a coil 32, a patient interface unit (PIU) 30, an electrode skin patches 38, an ablation energy generator 50, and a workstation 55. Note further each element and/or item of the system 10 is representative of one or more of that element and/or that item. The example of the system 10 shown in FIG. 1 can be modified to implement the embodiments disclosed herein. The disclosed embodiments can similarly be applied using other system components and settings. Additionally, the system 10 can include additional components, such as elements for sensing electrical activity, wired or wireless connectors, processing and display devices, or the like.

The system 10 includes multiple catheters 14, which are percutaneously inserted by the physician 24 through the patient's vascular system into a chamber or vascular structure of the heart 12. Typically, a delivery sheath catheter is inserted into the left or right atrium near a desired location in the heart 12. Thereafter, a plurality of catheters can be inserted into the delivery sheath catheter to arrive at the desired location. The plurality of catheters 14 may include catheters dedicated for sensing Intracardiac Electrogram (IEGM) signals, catheters dedicated for ablating, and/or catheters dedicated for both sensing and ablating. The example catheter 14 that is configured for sensing IEGM is illustrated herein. The physician 24 brings the distal tip 28 of the catheter 14 into contact with a heart wall for sensing a target site in the heart 12. For ablation, the physician 24 would similarly bring a distal end of an ablation catheter to a target site for ablating.

The catheter 14 is an exemplary catheter that includes at least one and preferably multiple electrodes 26 optionally distributed over a plurality of splines 22 at the distal tip 28 and configured to sense the IEGM signals. The catheter 14 may additionally include the sensor 29 embedded in or near the distal tip 28 for tracking position and orientation of the distal tip 28. Optionally and preferably, the position sensor 29 is a magnetic based position sensor including three magnetic coils for sensing three-dimensional (3D) position and orientation.

The sensor 29 (e.g., a position or a magnetic based position sensor) may be operated together with the location pad 25 including a plurality of magnetic coils 32 configured to generate magnetic fields in a predefined working volume. Real time position of the distal tip 28 of the catheter 14 may be tracked based on magnetic fields generated with the location pad 25 and sensed by the sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on the patient 23 to establish location reference for the location pad 25 as well as impedance-based tracking of the electrodes 26. For impedance-based tracking, electrical current is directed toward the electrodes 26 and sensed at the patches 38 (e.g., electrode skin patches) so that the location of each electrode can be triangulated via the patches 38. Details of the impedance-based location tracking technology are described in US Patent Nos. 7,536,218; 7,756,576; 7,848,787; 7,869,865; and 8,456,182, which are incorporated herein by reference.

The recorder 11 displays the electrograms 21 captured with the electrodes 18 (e.g., body surface electrocardiogram (ECG) electrodes) and intracardiac electrograms (IEGM) captured with the electrodes 26 of the catheter 14. The recorder 11 may include pacing capability for pacing the heart rhythm and/or may be electrically connected to a standalone pacer.

The system 10 may include the ablation energy generator 50 that is adapted to conduct ablative energy to the one or more of electrodes 26 at the distal tip 28 of the catheter 14 configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The PIU 30 is an interface configured to establish electrical communication between catheters, electrophysiological equipment, power supply and the workstation 55 for controlling operation of the system 10. Electrophysiological equipment of the system 10 may include for example, multiple catheters 14, the location pad 25, the body surface ECG electrodes 18, the electrode patches 38, the ablation energy generator 50, and the recorder 11. Optionally and preferably, the PIU 30 additionally includes processing capability for implementing real-time computations of location of the catheters and for performing ECG calculations.

The workstation 55 includes memory, processor unit with memory or storage with appropriate operating software loaded therein, and user interface capability. The workstation 55 may provide multiple functions, optionally including (1) modeling the endocardial anatomy in three-dimensions (3D) and rendering the model or anatomical map 20 for display on the display device 27, (2) displaying on the display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on the display device 27 sites of interest such as places where ablation energy has been applied. One commercial product embodying elements of the system 10 is available as the CARTO™ 3 System, available from Biosense Webster, Inc., 31A Technology Drive, Irvine, CA 92618.

For instance, the system 10 can be part of a surgical system (e.g., CARTO® system sold by Biosense Webster) that is configured to obtain biometric data (e.g., anatomical and electrical measurements of a patient's organ, such as the heart 12 and as described herein) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 12. Such locating may be done via an electrophysiological investigation during which electrical potentials are detected spatially resolved with a mapping catheter (e.g., the catheter 14) introduced into the chamber of the heart 12. This electrophysiological investigation, the so-called electro-anatomical mapping, thus provides 3D mapping data which can be displayed on the display device 27. In many cases, the mapping function and a treatment function (e.g., ablation) are provided by a single catheter or group of catheters such that the mapping catheter also operates as a treatment (e.g., ablation) catheter at the same time.

In another aspect, at least one single-axis magnetic sensor mounted on the catheter tip is configured to work in conjunction with at least one external magnetic sensor in a patient pad (i.e., under the patient). In one embodiment, there are three magnetic sensors mounted on the catheter that are oriented in three different directions (i.e., spaced 120 degrees apart) and that are configured to work in conjunction with three external magnetic sensors. Details of such technique are provided in the following documents: US Patent 5,391,199, US Patent 5,443,489, US Patent 5,558,091, US Patent 6,172,499, US Patent 6,177,792, US Patent 6,690,963, US Patent 6,788,967, and US Patent 6,892,091, which are each incorporated by reference as if fully set forth herein.

In another aspect, an impedance sensor on the catheter is provided that is configured to be used without any external sensors. Details of such technique are provided in the following documents: US Patent 5,944,022, US Patent 5,983,126, and US Patent 6,456,864, which are each incorporated by reference as if fully set forth herein. As used herein, the term sensor refers to any of the sensor arrangements described herein, whether the sensor is integrated with the catheter, or includes a combination of a sensor integrated with the catheter and additional sensors, such as external sensors.

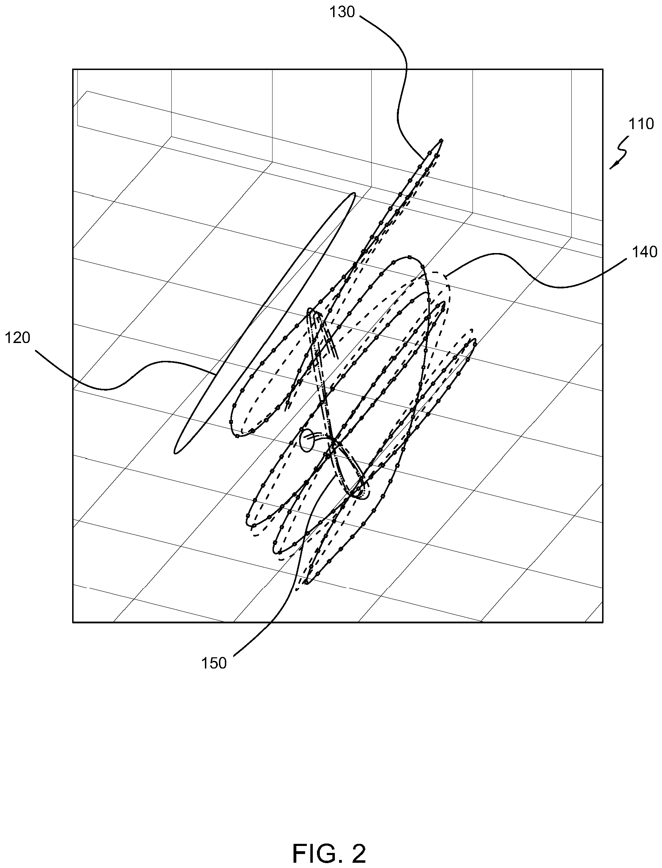

Regardless of the sensor arrangement, these sensors assist with modeling a patient's respiratory cycle, and identifying when a patient's lungs are breathing in or out. In one embodiment, when the lungs inflate or fill with air, then the impedance increases. The sensors can also generate a magnetic field, and this magnetic field can be used to detect the absolute position of the catheter 14. One skilled in the art would understand from the present disclosure that various methods and sensors can be used to determine a patient's respiratory cycle or the catheter's position. The respiration motion gathered from the sensors can be used to generate an ellipsoid (i.e., element 120 in FIG. 2) that provides a model of the respiration cycle.

FIG. 2 illustrates an exemplary image 110 including data regarding respiratory motion 120, catheter motion 130, reconstructed motion 140, and in-heart motion 150. This information is configured to be displayed on monitor 3. Each of these components is described in more detail herein.

The term respiratory motion 120 refers to motion that is based on respiration data, which can be gathered or collected in various ways. For example, in one embodiment, probes, sensors, or patches (such as sensors 18 in FIG. 1) are attached to a patient's body or sensors are integrated with the catheter 14 that may be used in conjunction with external sensors 18. This data reflects the changes in volume of a patient's lungs. The respiration motion 120 can be based on movement of a patient's diaphragm during breathing. The signal can be extracted by placing electrodes on a body surface. In one embodiment, the respiratory motion 120 is based on electrical signals between electrodes on a probe and patches on a body surface. In another aspect, the probes can measure displacement of one portion of the body relative to another portion of the body. This information is collected and further processed using the processes disclosed herein. In one aspect, a primary or major signal is generated by the sensors (such as sensors 18 in FIG. 1). This primary or major signal is then further processed and a derivative of the signal is determined. Respiration indicators and respiration vectors related to the respiratory motion 120 are described herein.

The catheter motion 130, which is also known as probe or sensor motion or original catheter motion, illustrated in FIG. 2 reflects data regarding an absolute position of the catheter 14. This data can be generated using any known tracking or sensing configuration for a catheter, probe, sensor, etc. In one aspect, a magnetic field and sensor 29 on catheter 14 is in turn used detect the position of the catheter 14. For example, catheter motion data can be obtained using magnetic transmitters external to a patient that generate signals based on a position of the catheter 14. In one aspect, the sensor configuration includes at least three magnetic sensors oriented in three different directions from each other that are mounted on a tip of the catheter and work in conjunction with a plurality of sensors that are external to the patient. In another aspect, an impedance sensor is provided and does not require any external sensors. The catheter motion data can be further processed via any combination of filters, smoothing, or post-acquisition signal processing.

The reconstructed motion 140 of FIG. 2 is a control element in one aspect. In other words, the reconstructed motion 140 is generated in order to provide a validity and error check whether there is an undesirable amount of difference between itself and the original catheter motion 130. In one aspect, the reconstructed motion 140 is equal to the respiration motion 120 plus the original catheter motion 30. An error variable, also referred to as ERR and described in more detail herein, can be determined based on the reconstructed motion 140 from the original catheter motion 130. Such determination based on the reconstructed motion 140 can be by subtraction or addition to the original catheter motion 130.

The in-heart motion 150 is also referred to as the catheter-heart motion or probe-cavity motion. In one aspect, the in-heart motion 150 is measured in millimeters, but can be measured used any metrics. This motion data can generically refer to any type of motion data for a probe, catheter, or sensor relative to any wall or chamber of a chamber. This data can ultimately be used to determine site stability, specifically relating to ablation in a patient's heart. The in-heart motion 150 is typically important for a surgeon due to this information indicating when the catheter is stable relative to the heart chamber wall. With the in-heart motion 150, it is possible to find a stability point, also known as a stability location or site. This information is important for determining various parameters regarding an ablation lesion, such as location, size, etc. During breathing, it is generally assumed that the catheter and the heart are moving up and down based on the diaphragm contracting. If the catheter is not stable relative to the heart wall or boundary (i.e., the catheter is not in contact with the heart wall or boundary) during ablation, then the ablation will likely not be successful or ablate the desired targeted location. In one aspect, the present disclosure is directed to providing more reliable and accurate mapping of in-heart motion 150 (i.e., catheter-heart motion or probe-cavity motion) based at least in part on respiration motion and other features, which addresses issues associated with any sudden movements occurring between end expiriums. In other words, by providing more accurate information regarding the relative position of the catheter and the cavity boundary, any movement that occurs between end expiriums can be accounted for by a surgeon and used to facilitate decisions regarding ablating procedures.

In one aspect, the systems, methods, and processes are based on the following three features: (1) signal processing, (2) respiration compensation, and (3) station finding or site stability location. One skilled in the art would understand that any one or more of these features can be used independent from the other features. For example, features (1) and (2) can be implemented without feature (3). In another aspect, feature (3) is specifically implemented in order to procedures involving the heart, and specifically involving ablation of specific areas of the heart. Any of the features described herein can used or adapted for determining aspects of chambers, and are not specifically limited to only being implemented with respect to heart chambers (i.e., navigation in the lungs).

In one aspect, signal processing includes converting respiration indicators (RI) into a main respiration signal and its derivative, i.e., respiration vectors (RV). The respiration data is gathered based on the sensors in one embodiment, which can either include a catheter integrated with a sensor, external sensors, or any combination thereof. In one aspect, before converting RI to RV, ablation effects are accounted for with ablation offset corrections, and low-pass filtering can be used to reduce heartbeat movement. In one aspect, the disclosed subject matter is based on decomposing catheter motion into two components: respiration motion and catheter-heart motion. Respiration motion is generally assumed to be correlated to RV via a correlation matrix A.

The catheter-heart motion is assumed to be a smoothed motion and can be modeled via a spline curve. Based on the foregoing, Equation 1 is provided:

$$\text{Measured Motion} = \text{Respiration Motion} (A \times RV) + \text{Catheter-Heart Motion} + \text{ERR}$$

(Equation 1)

Here, the correlation matrix A and the spline parameters are incrementally and simultaneously tuned in order to minimize root mean square (RMS) deviation between the measured motion after lower pass and the reconstructed motion in order to minimize the error.

In one aspect, the correlation matrix A is regressed in step n to match the respiration motion of step n-1, which is the total motion minus in-heart motion in step n-1. In other words, A is regressed in a current state to match the respiration motion of an earlier state, and the respiration motion is equal to the total motion minus the in-heart motion in the earlier state.

The correlation matrix A is a two-row by three-column data set. The correlation matrix A is configured in one aspect to transform two-dimensional RV into an ellipsoid in three-dimensional space. The matrix values are tuned or selected so as to adapt by multiplication the RV values to the respiration ellipsoid size (i.e., in mm). In one aspect, the RV values are relatively small (i.e., $1.e^{-3}$) and these are multiplied by large values (i.e., 3,000) in the A matrix to create movement (i.e., 3.0 mm movement) in the three-dimensional space.

Regarding the respiration motion, in one aspect, there are five RI channels. One skilled in the art would understand that the number of RI channels can vary. The RI generally indicate lung motion and changes in lung volume overtime. The RI generally each contain similar signals and noise. In one aspect, the RI are collected by attaching a plurality of probes directly to a patient. For example, six patches or probes can be attached to a patient. Electrical signals between the six patches or probes are measured and the RI are generated. The electrical signals may include voltage, resistance, and/or impedance. The RI corresponding to each patch or probe may be provided in mV in one embodiment.

Specifically, in one embodiment, RI are measures of the change in resistance (impedance) between patches, where the resistance is used via a temporal decomposition to extract the frequency band related to the respiration. The RI reflect the changes in the amount of air that flows into and exist in the lungs. The variation in airflow into and the air in the lungs may change the measurement of resistance between the patches (among the six patches).

Filters can be applied to the RI in order to remove noise or other elements. For example, a low pass finite impulse response (FIR) filter can be used to remove an imprint of the heartbeat on the RI signals. This step uses a 151-weight vector that is multiplied by 151 elements on each channel of raw data to produce one sample of filtered data, in one aspect. In other words, the filter multiplies the corresponding sample in a sliding window manner. One skilled in the art would understand that these parameters can vary.

The singular value decomposition (SVD) can be used to find the weights that select the most prominent respiration signal if multiplied by the measure five RI. This filtering step and processing step provides a smoother output signal and eliminates undesirable noise and disruptions caused by the heartbeat.

Based on the RI, a main respiration vector is extracted. In one aspect, this extraction is performed by calculating the first Eigenvector in SVD.

Generally, the SVD is configured to extract the most prominent common motion out of several similar respiration dependent signals. The common signal is found as a linear combination of the individual RI signals. In one aspect, to verify consistency of the linear weights, the SVD is done three times, including a first one on the first 30 seconds, a second one on the second 30 seconds, and third one on the entire 60 seconds of the RI buffer. In the event that the three sets of weights are similar enough, then the last set is selected. In other words, the similarity here refers to the V from the SVD. This V then becomes a weight vector, i.e., the RI2RV weights vector. In one aspect, the similarity parameter has a Pearson correlation of at least 0.5.

In one aspect, this process involves determining the first Eigenvector in the SVD of five RI signals over several respiration cycles. For example, a sixty second buffer can be used, which can correspond to ten respiration cycles. By using multiple cycles, it can be assured that the RI signals are accurate and reliable.

The disclosed subject matter also adds a phase shifted signal to provide ellipse-like motion or a model, shown by the estimated respiration motion 120 in FIG. 2. This phase shifted signal is computed by finding the derivative of the RI signals and combining the derivative using the same weights. This process is analogous to a phase shift between a Cos(x) function and Sin(x) function.

In one aspect, respiration vectors (RV) are produced based on the main respiration vector and a derivative of the main respiration vector. In other words, the five RI are processed using the steps disclosed herein to produce or determine two RV. Once these RV are determined, the following equation is provided:

$$\text{Measured Motion} = \text{Respiration Motion} (A \times RV) + \text{Catheter-Heart Motion} + ERR$$

$$(\text{Equation 2})$$

Equation 2 is generally similar to Equation 1, except Equation 2 uses RV instead of RI.

In order to determine the catheter location, raw location data regarding the catheter is filtered. In one aspect, the raw location data is filtered using the same low pass FIR filter that was applied to the RI signals to remove the heartbeat motion. As a result of the processing, synched and filtered respiration vectors and the catheter location are generated.

In summary, at the end of signal processing, the processes disclosed herein provides signals 120 and 130 in FIG. 2.

This feature generally includes estimating a correlation matrix A and spline parameters that represent the catheter's actual motion. The two RV are then transferred into a three-dimensional space.

Regarding the RV, the processes disclosed herein generally transfer this 2D vector into a 3D ellipse or ellipsoid in 3D space, as shown by element 120 in FIG. 2. The ellipse drawn in FIG. 2 can be oriented in any direction, and the main purpose of compensating for respiration involves modeling movement of a patient's breathing on the catheter or probe.

The correlation matrix A (also referred to as the transformation matrix) converts the two-dimensional vector into a three-dimensional ellipsoid. The correlation matrix A is determined based on regression, in one aspect. The root mean square error (RMSE) is minimized between the modeled motion (i.e., respiration motion + catheter motion) and the measured motion to provide the correlation matrix A.

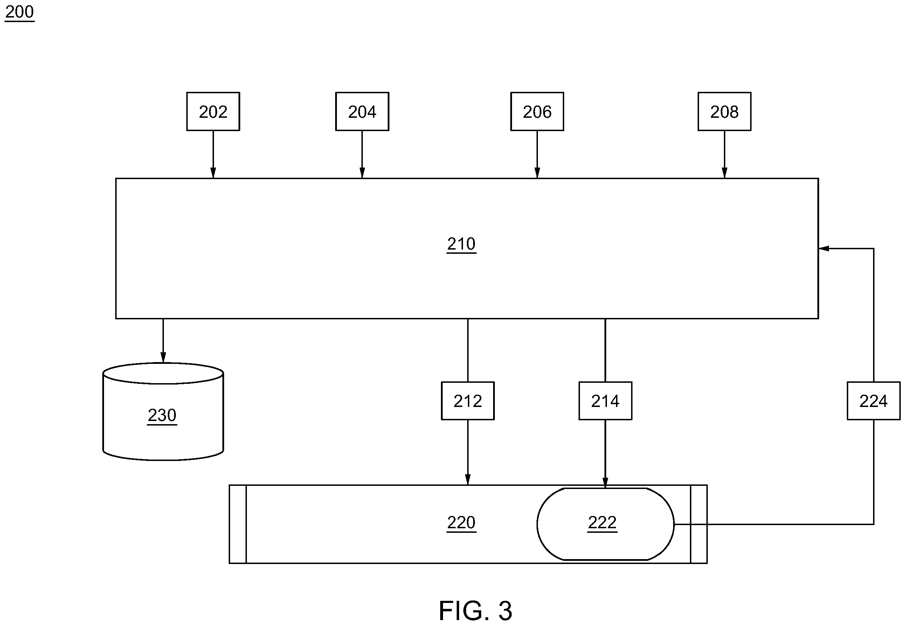

As shown in FIG. 3, a system 200 is disclosed that includes a site stability module 210. This system 200 can be implemented, integrated, or configured to interface with the computing system 4 shown in FIG. 1. The term module, as used herein, can refer to any computational component or interface, and can include any hardware component or software component.

The site stability module 210 can include any one or more of the features disclosed herein. In one aspect, the site stability module 210 is configured to receive data from various inputs. For example, location data 202, respiration indicator data 204, status data 206 (such as location status and status of the RI), and initialization or algorithm parameters 208 can each be provided to the site stability module 210. In one embodiment, the data is provided streaming in 16.67 ms intervals. One skilled in the art will understand based on this disclosure that the data steaming parameters and cycles or periods can vary.

From the site stability module 210, session stability time segments 212 (i.e., start-end segments) and the catheter-heart location data 214 are transmitted to a central module 220. In other words, the site stability module 210 provides output parameters that include the last stability segment parameter and parameters of the last set of sites in the last ablation session. In one aspect, stability is a segment of duration of three seconds or greater in which the catheter-heart speed is below a certain speed threshold. The site is part of the stable segment during ablation time, and a segment of stability during ablation can be divided into several sites.

In one embodiment, the central module 220 can include any centralized computing system configured to receive, process, and/or transmit data to and from the site stability module 210. In one example, the central module 220 is a CARTO VISITAG™ processing unit. One skilled in the art would understand that various types of configurations can be provided that are in communication with the site stability module 210.

At the central module 220, compensated location data 222 for each session can be saved and stored. This data 222 can be further processed, filtered, etc. at the site stability module 210. A recordation module 230 can optionally be provided. The recordation module 230 can be configured to assist with debugging and post analysis of collected data. Element 224 in FIG. 3 represents a recalculation step in which information from the central module 220 is continuously provided back to the site stability module 210 in order to refine and recalculate information regarding site stability.

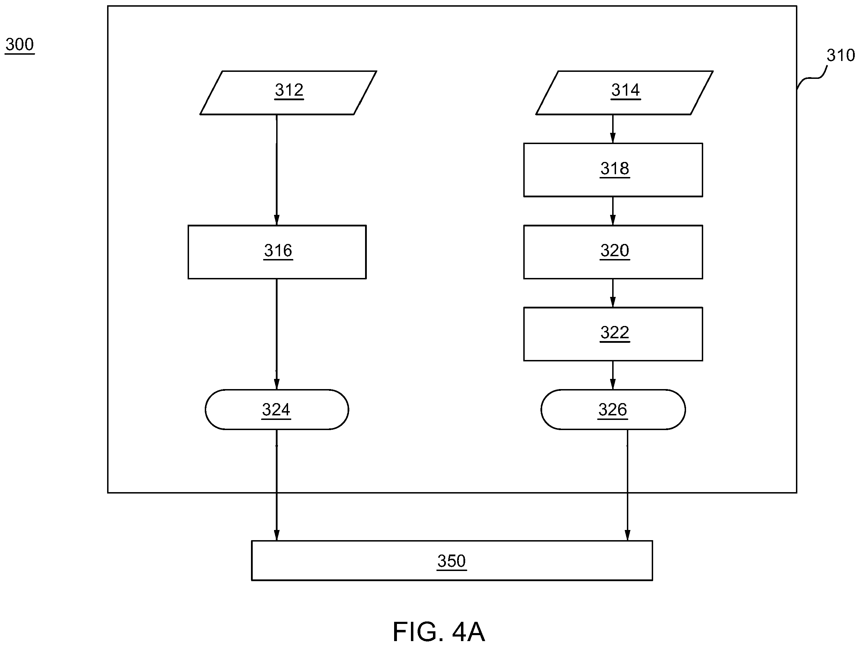

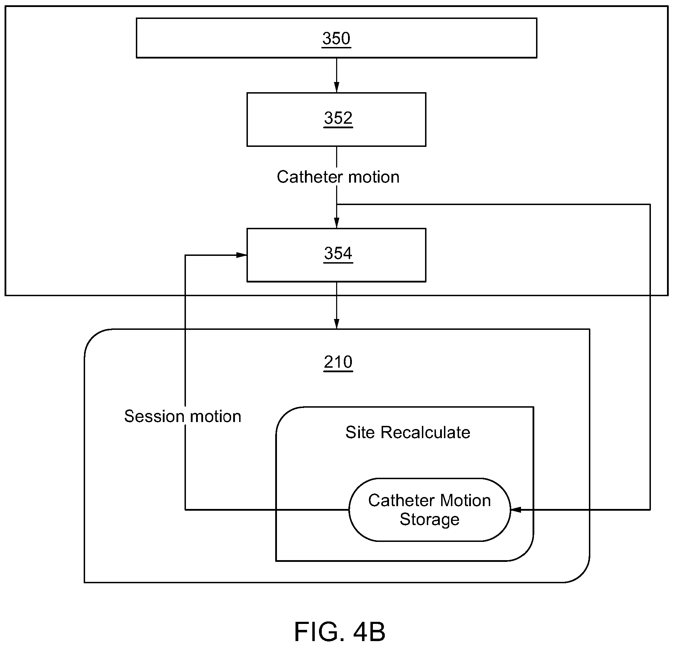

Referring to FIGs. 4A and 4B, a flow diagram is provided to illustrate various steps of process 300 for providing site stability. As shown in FIGs. 4A and 4B, the process 300 generally includes two distinct phases for site stability, a pre-processing phase 310 and a stability detection phase 330. Preprocessing phase 310 is the stage generally where the raw signals from the measurements are filtered and transformed providing a filtered catheter motion vector and a respiration vector. The filtered catheter motion may still include a respiration factor. Decomposition 350 which is during the stability detection phase 330 is where the filtered catheter motion is separated into respiration motion and respirator free catheter motion. Stability detection phase 330 is the stage in which sliding motion is used to detect ablations stations inside stable segments during an ablation. Preprocessing phase 310, decomposition 350 and stability detection phase 330 are described in more specific detail below.

In the pre-processing phase 310, location data 312 and the RI 314 is provided. In other words, the pre-processing phase 310 includes at least two groups of channels: the location channels, and the RI channels. This data can be set or generated in relatively small increments. For example, the data can be set at least every 16.67 ms.

The location data 312 is further processed. For example, the location data 312 can be processed by applying a low pass filter or low pass FIR filter at step 316. After this step, the filtered location, i.e., the last filtered location, is determined at step 324.

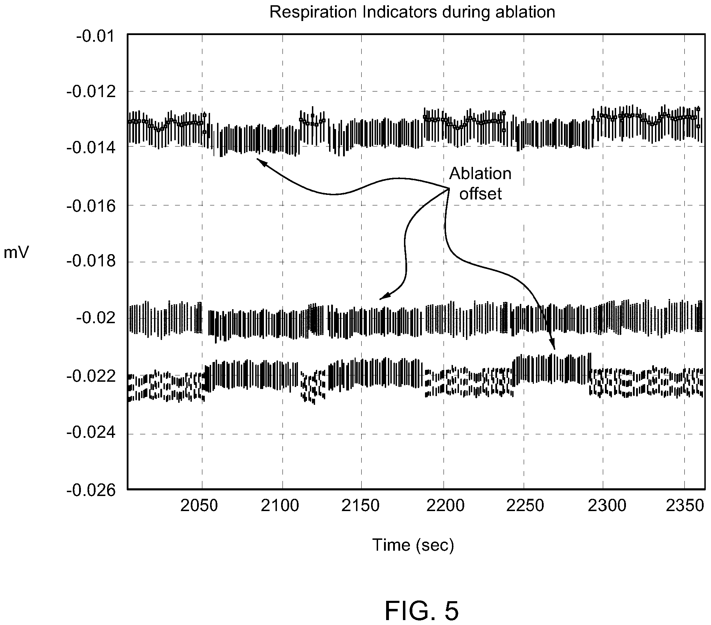

The RI 314 can also be further processed through a series of steps or processes 318, 320, and 322. As shown in FIG. 3A, the RI 314 can first be analyzed for ablation corrections at step 318, which accounts for any offset caused by the ablator. The offset from the ablator affects the ability to estimate respiration motion. Accordingly, reducing the effect of the offset provides an accurate output regarding the RI 314. In one aspect, an ablation offset correction factor is determined by measuring RI signals prior to ablating and during ablating. Whenever the ablator is turned on, then the estimated offset per channel is compensated (e.g., subtracted or added) from the RI signals. The offset is estimated based on the RI signal change between the RI signals measured just before ablation starts to the RI signals measured just after the ablator start. The effect of the ablation offset is shown in FIG. 5, which illustrates an ablation offset by the shift in the RI during ablation.

Returning to FIG. 4A, the data is filtered at step 320. In one embodiment, the filters include a low pass filter and a derivative filter. In one embodiment, the low-pass filter and derivative filters are configured as FIR filters with 151 sample kernel. In one aspect, preprocessing includes the following steps. First, the original five RI channels are obtained. Second, ablation offset correction is performed. Third, low-pass and derivative pass filters are performed to create filtered respiration indicators (FRI) and derivative respiration indicators (DRI) using a sliding window of 151 samples of FIR filter. Fourth, the SDV is performed to find the most prominent weights. Fifth, the most prominent weights are implemented on the FRI to obtain RV(1) and on the DRI to obtain RV(2). One skilled in the art would understand that additional filters can be used and additional steps may be implemented.

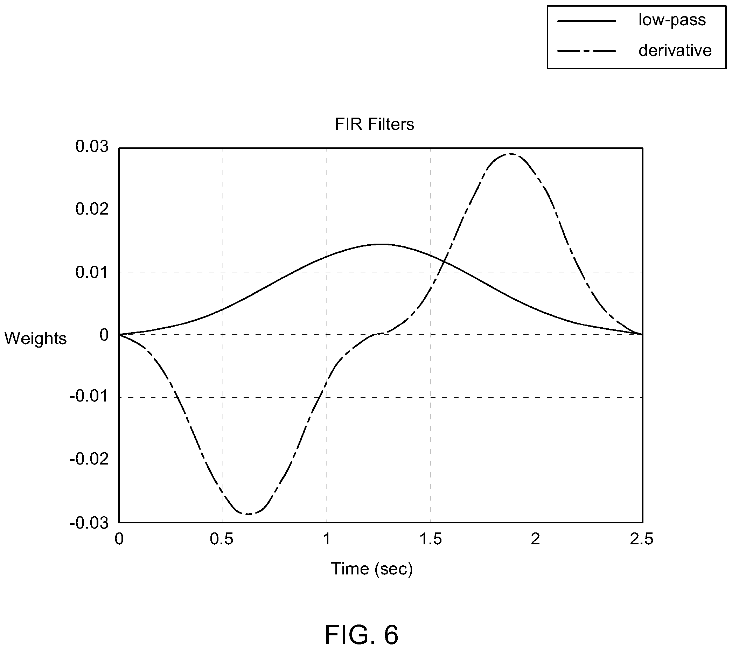

FIG. 6 illustrates one example of filters that can be used at step 320, and illustrates the weights for a low pass filter and for its derivative signal. As shown in FIG. 6, different weighted factors are applied over time for the low-pass filter and the derivative filter.

The data regarding the RI 314 is further processed at step 322 by calculating the first Eigenvector of the RI 314 and by calculating the derivative of the first Eigenvector of the RI 314. The SVD is used to find the first Eigenvector of the filtered RI in order to reduce redundancy and noise. The mean compensated (e.g., subtracted or added) filtered respiration indicators (FRI) and the derivative respiration indicators (DRI) can be stored. In one embodiment, this information is stored in one-minute buffers. One skilled in the art would understand that the buffer duration can vary.

In one aspect, the SVD is only performed once at the beginning of a predetermined period when the buffers are full with a valid signal. The SVD produces five coefficients of a first Eigenvector (V1), and the five coefficients are used to produce the respiration vectors (RV) at step 326. In one embodiment, the RV equal is equal to FRI x V1, DRI x V1.

At the end of the pre-processing phase 310, the last minute of the RV data (produced at step 326) and the filtered location data produced at step 324, are synchronized and stored in the last-minute buffer.

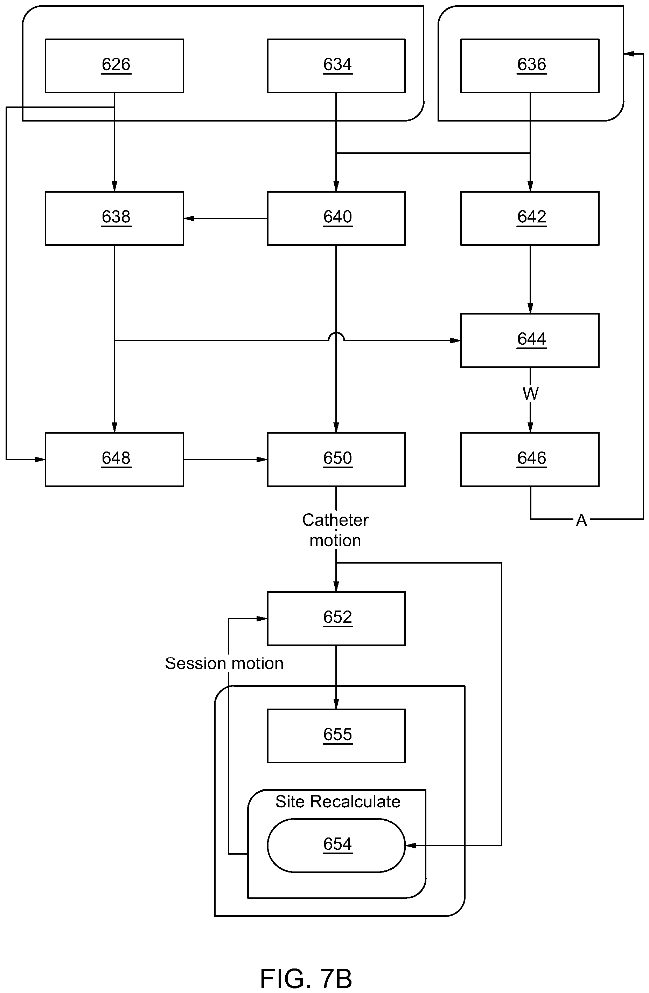

Data from steps 324 and 326 is provided to the stability detection phase 350. During the stability detection phase 350 (shown in FIG. 4A bottom and FIG. 4B at the top) motion decomposition 352 is carried out. More detail regarding motion decomposition is provided by FIG. 7B. From motion decomposition 352, the process includes calculating site stability and sites 354. This step provides data back to a site stability module 210, which is configured to receive catheter motion data and provide session motion data. Information regarding catheter motion and site information is stored with the site stability module 210. Site stability parameters can be recalculated based on the information provided to the site stability module 210.

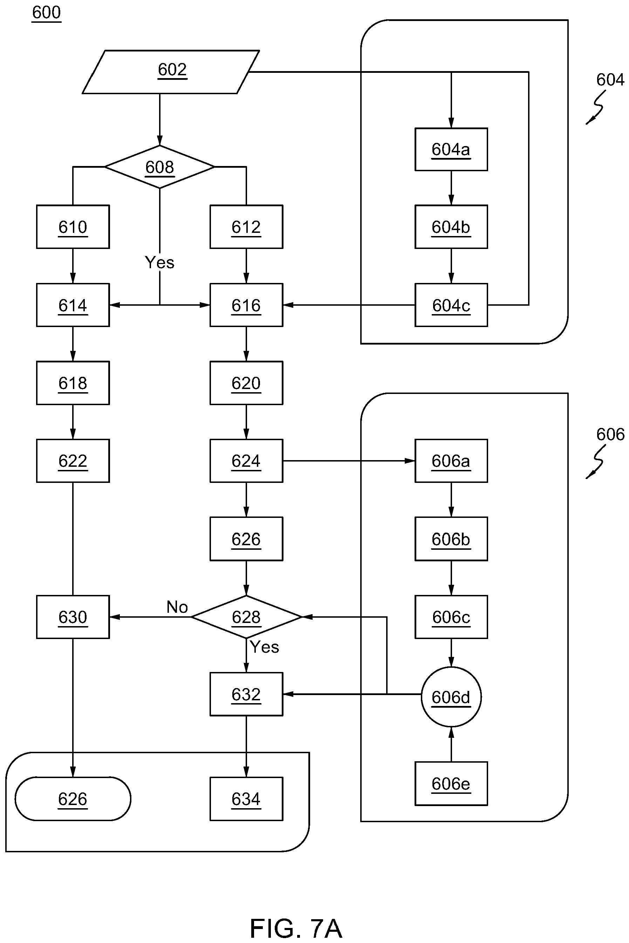

A more detailed overview of the pre-processing phase 310 is illustrated in FIGs. 7A and 7B. As shown in the process 600, a pre-processing phase starts with obtaining a data set, i.e., step 602. The data set can include the RI, locations, statuses and various other information.

As shown on the right side of FIG. 7A, an ablation correction phase 604 is provided. This phase is configured to correct the offset in the RI signals due to ablation interference. The ablation correction phase 604 includes setting a buffer at step 604a. Next, at step 604b, the offset is estimated per channel by comparing the RI signal edges (i.e., signal 5 and 95 percentiles) just before and just after the ablator is turned on. Then, at step 604c, this estimated offset is compensated (e.g., subtracted or added) from the RI signals. In one aspect, ablation offset is measured and corrected per ablation session, and is performed dynamically and continuously. With each ablation, the estimation becomes more accurate due to the offset measurements being averaged over time.

As shown in FIG. 7A, the buffer at step 604a can include a 1.5 second buffer of the RI signals to enable fast estimation. The buffer can include at least 151 samples of RI signals. The parameters for accounting for the effect of ablation on the RI signals can vary depending on specific circumstances, such as the number of RI channel signals, ablation strength and duration, characteristics of the patient, etc. Data from step 604c is ultimately sent to step 616, described in more detail below.

Continuing from step 602, a validation step 608 is carried out to determine if the data set is complete. In one aspect, the computing system 4 or a manager in a software module confirms the validity of the data. As used herein, the term manager can refer to any computational interface, such as an interface within CARTO® VISITAG™ processing unit or central module 220. This component is configured to insert or input data and to also receive an output of data. In the event that the data is invalid, the manager sends a reset or soft-reset signal to the algorithm processor to empty all of the buffers and begin filling the buffers again with new data if errors are detected. The algorithm ensures that the buffers are full with valid data before progressing to a subsequent step.

If the data is determined to not be valid due to location data issues, then the buffer is reset at step 610, and location filter buffers are updated at step 614. In one embodiment, step 614 includes 151 × 3 samples.

If the data is determined to not be valid due to RI channel related issues, then the buffer is reset at step 612, and RI filter buffers are updated at step 616. In one embodiment, step 616 includes 151 × 5 samples.

If the data is determined to be valid, then the location filter buffers and the RI filter buffers are also updated, as described with respect to steps 614 and 616, but the buffers do not need to be reset.

Continuing on from steps 614 and 616, the location data and the RI signal data both undergo a filtering step. The location data from step 614 is filtered at step 618. In one embodiment, the filter at step 618 is a low-pass FIR filter. The RI signal data from step 616 is also processed. In one embodiment, the filter at step 612 is a low-pass and derivative FIR filter.

Step 622, which follows step 618, then updates location buffers. Step 624 follows step 620 and includes updating RI buffers. From step 622, the location data is further processed at step 626, which includes obtaining a last-minute buffer, and obtaining filtered catheter motion.

Regarding the RI signal data, after step 624, this data is further processed using an Eigenvector estimation phase 606. In general, phase 606 includes transforming every sample in the 5D FRI vectors into their first Eigenvector using the Eigenvector coefficients in vector V. In one aspect, vector V is a five-element vector of weights that multiple the five RIs to obtain the most prominent respiration vector. The vector V is obtained via SVD decomposition and RI = U*S*V' in one aspect.

Step 606a includes measuring breaks in the derivative RI (DRI) signal. Step 606b includes checking that there is no respiration gap and the data is valid. Step 606c includes calculating V using the SVD. The SVD is calculated only once the FRI buffer is full with valid numbers and no respiration gap was found. In one embodiment, this step can include calculating V in one second intervals. Once V is obtained at step 606d, then the data can be further processed at steps 628 and 632. Step 606e is a validity check, and if a soft-reset signal was received, then vector V is reset to zero.

Step 628 includes checking whether (V1) > 0. If not, then step 630 includes waiting for the next round of data. If so, then step 632 defines the RV = [FRI x V, DRI x V]. Calculating V (or RI2RV as used in one aspect of the algorithm) includes the following steps. Once the RV buffers are full, a two second test can be performed to determine if there are any respiration gaps (i.e., due to apnea) inside the DRI buffer. If the buffers are full and there are no gaps, then a calculation is carried to out to determine three different V by three different SVDs: one on the first 30 seconds, one on the last 30 seconds, and one on the entire 60 seconds DRI buffer. If all the three Vs are correlated (i.e., within a predetermined value of each other), then the 60 second V is selected as the new V going forward. The process then verifies that the minima of the resulting respiration signal RV(1) is correlated to a maxima of the y-location of the probe. In one aspect, the minima of the respiration signal (RV1) is preferred to represent the end-expirium (as compared to the in-expirium). For this process, the corresponding Y-values of the catheter location are checked and the process ensures that the Y values in the respiration minima is larger than the Y values in the respiration maxima because the Y value is maximal in humans at end-expirium times. In this orientation, the Y-direction in is oriented in a heart-head direction in a patient as the patient lays on their back.

If the resulting respiration signal RV(1) is not correlated to the maxima of a y-location of the probe, then the selected V is reversed to be negative (i.e., -V). Finally, step 634 includes the last-minute buffer that was used in step 626, to provide RV.

As shown at the top of FIG. 7B (included in FIG. 7A to provide overlapping reference), filtered motion is provided from step 626 and the RV are provided from step 634. Step 636 includes providing a correlation matrix A, which accounts for weights buffering and respiration gaps. Generally, FIG. 7B shows the steps for motion decomposition according to one aspect. The motion decomposition assumes that the filtered catheter motion (FCM) can be modeled as a sum of two main components: (1) Respiration compensation vector (RCV) = Correlation matrix A x RV; and (2) Catheter-heart motion = Smooth(FCM - RCV) = Spline(FCM - RCV).

The term "smooth(x)" as used herein generally refers to a mathematical smoothing function, which can be implemented according to any computer interface, electronics, or programming tool, such as MATLAB®. Various other functions, such as "smooth," "exp," "pinv," "spline," "rms," "norm," etc., used herein may be implemented using any known computer interface, electronics, or programming tool, such as MATLAB®. These computer interfaces, electronics, or programming tools are generally configured to provide matrix, array, and data manipulation, plotting functions, data implementation, and/or algorithm implementation.

The term "smooth" as used with respect to functions or equations herein refers to a method of noise reduction or a data set in one aspect. In one aspect, the smoothing function uses a moving average, a filter, or uses a smoothing spline.

In one aspect, the term "spline(x)" as used with respect to functions or equations herein refers to a function that calculates, returns, or generates a vector having interpolated values that correspond to points of an array, matrix, or dataset x. In one aspect, a cubic spline interpolation can be used.

In one aspect, the term "exp(x)" as used with respect to functions or equations herein refers to an exponential function $e^x$ and determines each element in an array, matrix, or dataset x.

In one aspect, the term "pinv(x)" as used with respect to functions or equations herein refers to a function that calculates, returns, or generates a Moore-Penrose Pseudoinverse of an array, dataset, or matrix x.

In one aspect, the term "norm(x)" as used with respect to functions or equations herein refers to a function that determines a scalar or magnitude value, and calculates, returns, or generates a measure of magnitude of elements in an array, matrix, or dataset x.

In one aspect, the term "max(x)" as used with respect to functions or equations herein refers to a function that calculates, returns, or generates the maximum elements of an array, matrix, or dataset x.

In one aspect, the term "rms(x)" as used with respect to functions or equations herein refers to a function that calculates, returns, or generates the root-mean-square of an array, matrix, or dataset x.

Any one or more of the function terms used herein can be implemented using any computational element or software program configured to perform computational analysis and functions. One such example of a program is MATLAB®. One skilled in the art would understand these mathematical or programming functions, and equivalents or variants thereof, can be implemented to carry out specific functions, calculations, or manipulations of the data, matrices, arrays, and information described herein. Any of the functions described herein can be implemented on the computing system 4, or any other electrical or computer hardware and software.

Decomposition is calculated every 100 ms in one embodiment. These 100 ms segments can be calculated on the entire last minute of data in order to allow for slow adaption of the correlation matrix A over the last minute.

In one aspect, two versions of the catheter-heart motion are used by controlling the curvature of the spline. In one version, intermediate catheter motion is calculated with a single curvature or smoothing parameter. Step 638 includes estimating the intermediate catheter motion with the following calculation: Smooth(FCM-RCV). This smoothing parameter limits the speed of the spline direction change and this is equivalent to assume slow accelerations. The intermediate catheter motion is used in the estimation of the correlation matrix A. Specifically, the correlation matrix A is tuned using segments with slow intermediate catheter motion speed (i.e., slow segments). On these slow segments, the catheter acceleration is small with respect to the respiration motion, and the correlation matrix (A) is estimated more accurately. During step 638, the intermediate catheter motion is estimated using a smoothing function to limit the resulting curvature.

In one aspect, intermediate catheter motion (ICM) = Smooth(T0,(FCM-RCV),SmoothP). In this equation, T0 is a vector of reference time (0 to 60 seconds in 0.1 second increments). The inaccuracy is estimated by the norm function of the estimation error. Using step 638, the ICM has an error component (ICMerr) and ICMerr = Norm(FCM-RCV-ICM), and SmoothV = Exp(-2*ICMerr), and SmoothP = 0.005. This process corrects or updates the ICM to allow larger curvature where the ICMerr is unacceptably large. If ICM were equal to FCM-RCV, then the ICMerr would be zero.

SmoothP is a smoothing parameter that limits the spline curves second derivative (i.e., acceleration), and is generally defined as a smoothing parameter to control the curvature through a segment. SmoothV is generally defined as smoothing vector to control curvature per data point. During step 638, it is assumed that slow accelerations help converge the correlation matrix A. The slow accelerations, i.e., the intermediate catheter motion (ICM), are used in combination with faster accelerations, i.e., the catheter motion (CM), in order to provide a more robust and accurate reconstruction. The ICMerr or error of the ICM is an estimation error and this is equal to Norm(FCM-RCV-ICM). The ICMerr is used to estimate the accuracy of the reconstruction of the FCM. Based on this, the spline is corrected when the error of slow motion is high. In one aspect, the smoothing function smooths data using a moving average filter.

In another aspect, shown in step 648, catheter motion (CM) is the corrected ICM using the correction vector (SmoothV) and allows fast acceleration where the intermediate catheter motion is determined to be inaccurate. This correction vector (SmoothV) allows different curvatures (i.e., accelerations) at different spots along the last minute of data. The CM equals: Smooth(T0, (FCM-RCV), SmoothP, smoothV). This process is a way to force the smooth function to be more flexible, which allows for larger curvature in points where the reconstructed error is larger.

Step 650 includes estimating the catheter motion based on the following equation: Smooth(FCM - RCV,SmoothV). In one aspect, a function or process is carried out to smooth the catheter motion with a spline model by limiting the spline curvature (i.e., the second derivative of the curvature).

When estimating the CM, fast segments are permitted to have increased curvature to provide for more accurate and better reconstruction. The smoothing spline function f is shown in the following equation:

$$smoothP \cdot \sum_{j=1}^{n} \left| xyz_j - f_j \right|^2 + (1 - smoothP) \cdot \int smoothV(t) \left| D^2 f(t) \right|^2 \, \mathrm{dt}$$

(Equation 3)

In this equation, $|z|2$ represents the sum of the squares of all the entries of z, n is the number of entries of x, and the integral is over the smallest interval containing all the entries of x. The default value for the weight vector W in the error measure is ones(size(x)). The default value for the piecewise constant weight function smoothV in the roughness measure is the constant function 1.

In Equation 3, $D^2 f$ denotes the second derivative of the function f. The parameter smoothP controls the smoothing strength. When smoothP = 1, the spline goes through all the original data points without any curvature limitations. When smoothP = 0 the spline cannot include any curvature so the result is a straight line. In this situation, smoothing can be used to control the catheter allowed acceleration.

For intermediate catheter motion (ICM) only the non-zero smoothP parameter is used, and the sharp movements are smoothed or filtered out. To model fast or sharp movements, the smoothV vectors having a value less than 1 are used. SmoothV values less than 1 put extra weight or emphasis on certain aspects and therefore can include higher curvature in those places.

The whole concept of smoothing in this aspect is balancing between two contradictory elements. The first element generally tries to remain true or accurate to an input vector even when there is noise (i.e., many inconsistent direction changes). The second element is to be as smooth as possible with minimal direction and speed changes. SmoothV is a vector of values that directs the smooth function to prefer the first element over the other, or vice versa, in different parts of the input vector. So as SmoothV values are closer to 0, the smoothing function should be more accurate and less smooth. In one aspect, smoothV allows smoothP in Equation 3 to be localized.

From step 650, catheter motion is used for site segment detection at step 652. At step 654, catheter motion and site segment detection data are sent to a central module 655 that is configured to store data regarding the site segment and catheter motion. An iterative process is then carried out that analyzes session motion, and recalculates the ablation site. The ablation is divided into sites and is recalculated in every step, and can vary significantly according to compensated catheter-heart motion. This catheter compensated location can be saved in a central module or storage unit for site recalculation after a user or surgeon has adjusted various control parameters.

Step 640 includes calculating the RCV in the last minute of information and data. In one embodiment, the RCV = A x RV. In one aspect, correlation matrix A is also identified as RV2RCV. Estimating the correlation matrix A is generally performed using weighted mean squares and matrix inversion according to one embodiment. The weights (W) used in these calculations reflect how each data point in the last minute is configured to estimate the correlation matrix A according to at least one of the following criteria: (i) weight decay with time, (ii) preference for slow intermediate catheter motion speeds, (iii) RV validity, and (iv) duration of time from ablation.

Regarding factor (i), older samples generally receive lower weights than recent samples. Regarding factor (ii), slower speeds on long segments receive higher weights. Regarding factor (iii), during apnea, the RV is reduced to very close to zero, and cannot be used in estimating the correlation matrix A. Accordingly, apnea segments receive zero weights. Apnea detection occurs at step 642. Apnea is also generically known as depth of breathing, or the shallowness of breathing. Factor (iv) results in recent ablation segments receiving higher weights. The weight vectors are identified as the stability weights, and the step of estimating the stability weights is generally shown as step 644 in FIG. 7B. In other words, the intermediate catheter motion from step 638 is used to estimate the stability weights in step 644.

The apnea detection associated with factor (iii) and step 642 is defined in more detail. Respiration apneas are segments of time in which the respiration signal, i.e., the RV, is reduced due to apnea or other forms of shallow respiration. Due to their influence, the correlation matrix A should generally not be estimated during these segments. By using weight associated with each sample (which includes RV and location data), the effects of apnea or shallow respiration are minimized. Weights during shallow respiration (i.e., small RV signals) are zeroed, which eliminates the impact of these measurements on the overall estimation and correlation matrix A.

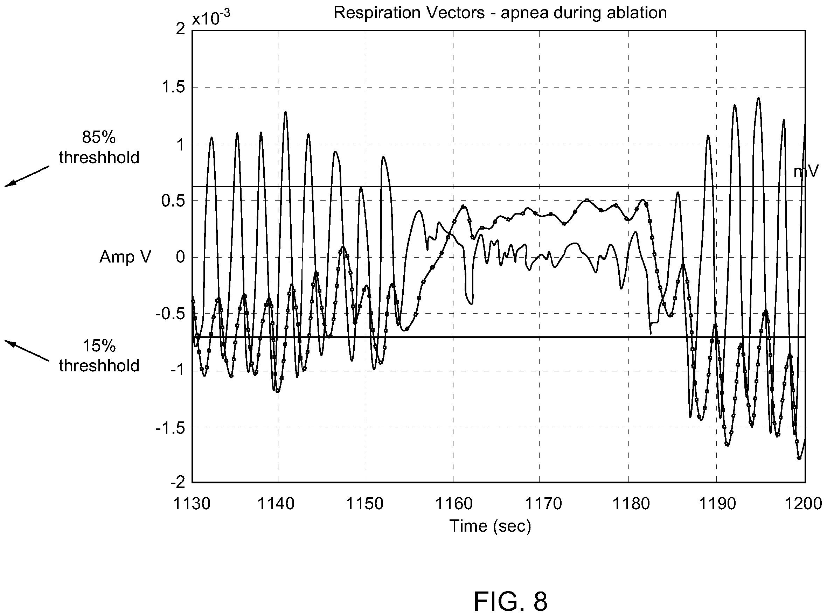

FIG. 8 illustrates the effect of apnea on the RV during ablation. A gap in respiration is generally defined as time segments in which the respiration derivative cannot reach a predetermined extreme value and can only reach below 85% of the predetermined extreme value. As used in this context, these small and large parameters are defined by analyzing the 15 - 85 percentiles, and ignoring signals above and below these percentiles. Consecutive samples that are within the 15-85 percentiles are counted and analyzed. If the signal in a continuous segment happens to occur inside these thresholds and not crossing them, then the segment of all the samples in this continuous segment is indicated as a respiration gap, i.e., a shallow breath, and therefore can be discarded. The percentile values are selected over the last minute of data and define thresholds for the respiration derivative amplitude. When the respiration derivative signal is between the thresholds for a predetermined period, such as 5 seconds (which corresponds roughly to one respiration cycle), then the respiration signal is considered too weak and therefore should be discarded. The percentile values can be modified and generally selected to identify shallow respiration. One skilled in the art would understand that other filtering or analysis can be used to identify shallow respiration.

Further details regarding estimating stability weights are provided herein. Generally, the decomposition process into respiratory related motion and catheter related motion is performed more accurately when the catheter is relatively stable or is slowly sliding against another surface, i.e., tissue. Identifying relatively stable segments is important for producing a reliable output. Stable segments are generally defined herein as segments of time in which the intermediate catheter motion speed is relatively slow (i.e., < 2.5 mm/sec) for a specific period of time (i.e., > 3-5 seconds). Estimating the correlation matrix A is carried out using the stable segments or essentially stable segments. Stable segments are emphasized due to the assignment of higher weights for samples during stable times. Stability weights are converted into a vector of numbers that correspond to the last minute and represents the point relevancy for the correlation matrix A estimation (i.e., the RV2RCV matrix). Besides speed stability, factors regarding recency (i.e., the age or how old a segment is) and data segments collected during ablation are also considered. Recent samples receive higher weights than older samples, and ablation segments receive higher weights than non-ablation segments.

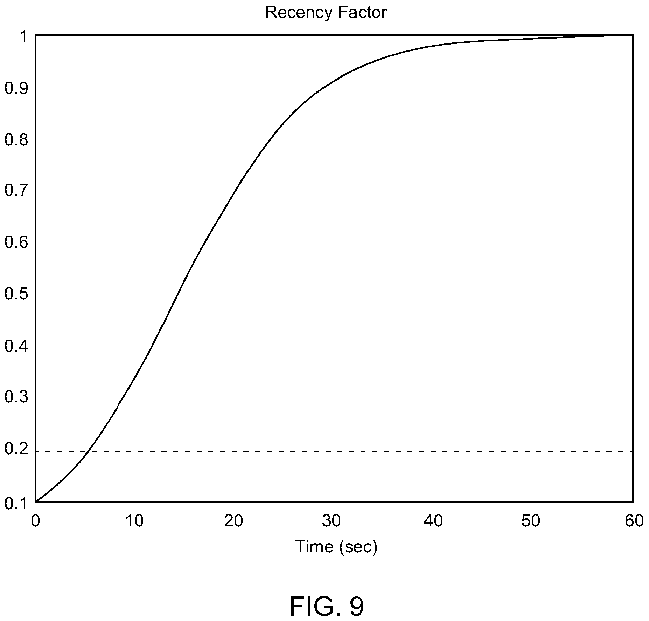

FIG. 9 illustrates one aspect of the recency factor with respect to stability weights. As shown in FIG. 9, higher weights are assigned to more recent samples (i.e., the right-hand side of the graph) and the older samples receive a lower weight. As shown in FIG. 9, a sigmoid provides a smooth transition between recent and older signals.

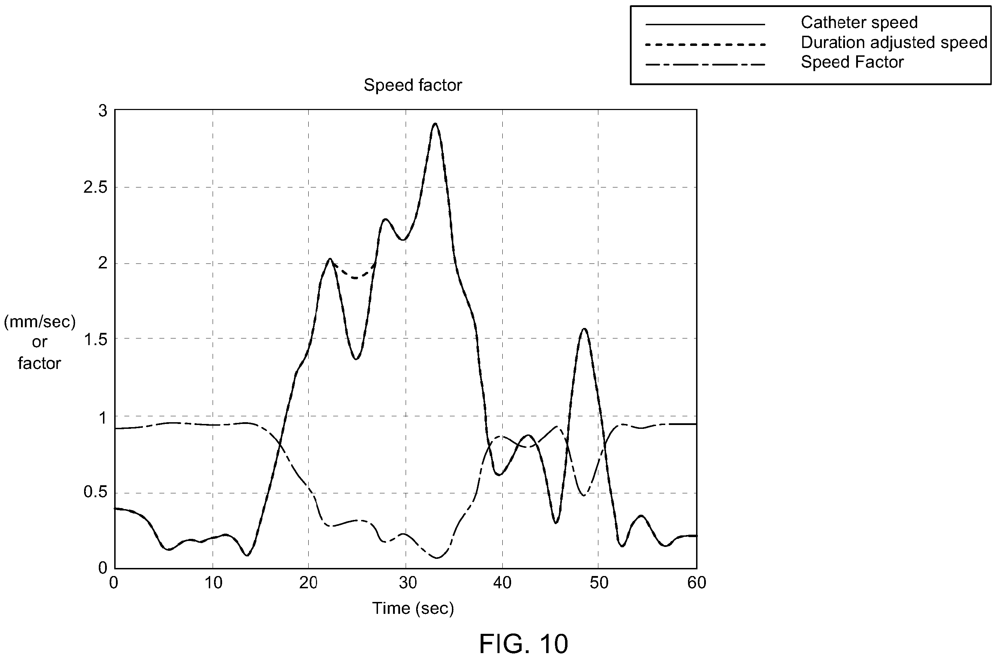

FIG. 10 illustrates one aspect of the speed factor with respect to stability weights. As shown in FIG. 10, higher weights are used when the catheter estimated speed is low, and lower weights are used when the catheter estimated speed is high. In one aspect, the weights are decreased when the low speed duration is shorter than an average respiration cycle. As shown in FIG. 10, the duration is adjusted by artificially increasing the estimated speed.

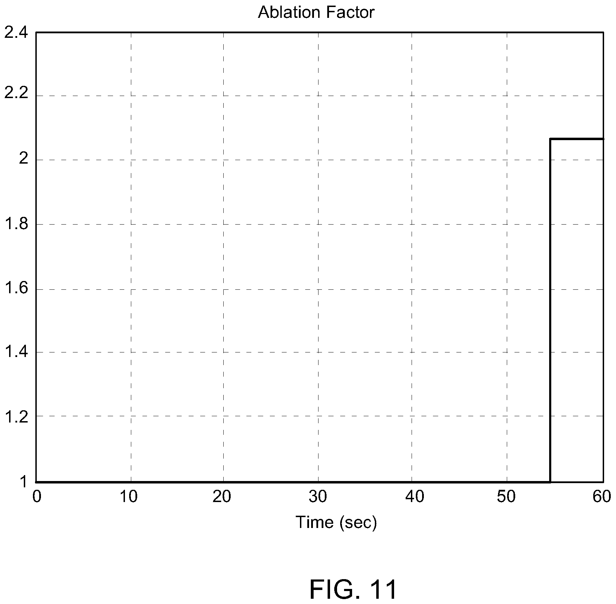

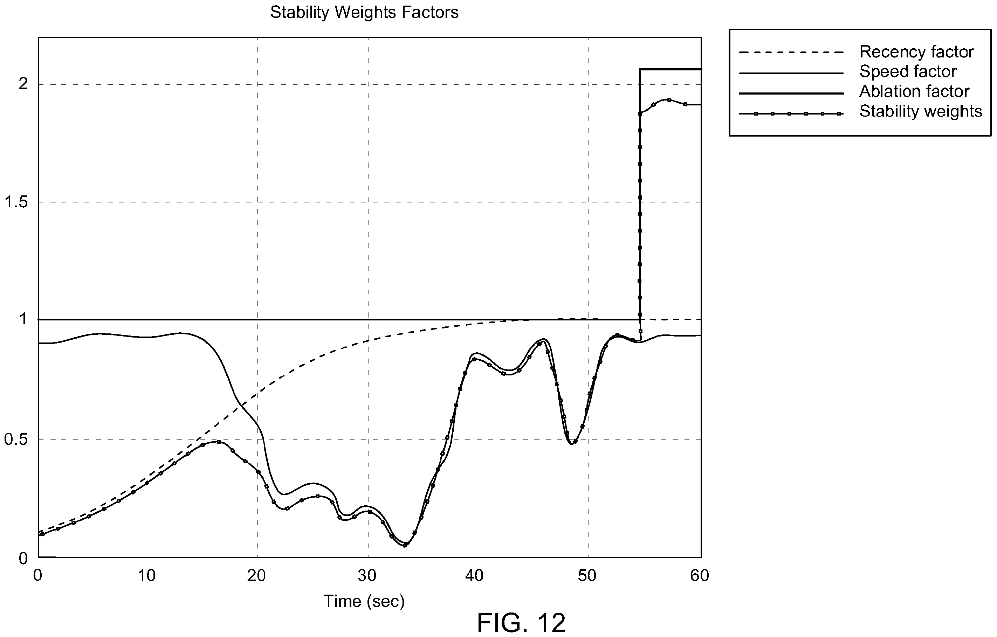

FIG. 11 illustrates one aspect of the ablation factor with respect to stability weights. In general, higher weights are assigned to signals when ablation is occurring, as shown in FIG. 12. In one aspect, the three factors described above with respect to FIGs. 9, 10, and 11 are used to generate the total stability weights. In other words, the total stability weights vector can be a sample by sample product of all three factors. One skilled in the art would understand that more than three factors can be used. FIG. 12 illustrates how the stability weight factors can be measured over time. In one aspect, the recency factor is only calculated at the initialization because this factor is fixed.

Returning to FIG. 7B, using the weights (W), the correlation matrix A is estimated at step 646 using the following equation: A(2x3)=Pinv(RV) x W(FCM-RCV). As used in this aspect, A(2x3) transforms the RV (two columns matrix representing the respiration signal and its derivative) into the RCV (three column matrix representing the X-Y-Z of the respiration matrix). Matrix A is the transformation matrix between the respiration vectors (RV) and a 3D respiration ellipse. This equation represents a regression using mean square since FCM = A*RV + CM + err, which represents a decomposition equation. Because FCM cannot be decomposed perfectly into these models, there will be some error (err) which represents the difference between the FCM or actual motion and the sum of the two models. Essentially, these processes tune the transformation matrix A to minimize the error. In order to minimize the error, the correlation matrix A is regressed such that A = ((FCM-(CM))/RV which equals Pinv(RV) * (FCM-(CM)). With the weights W added to give greater weight to time segments with a slow (CM), the equation then becomes A = Pinv(W*RV)*W*(FCM-(CM)), in one aspect.

As shown in FIG. 7B, the correlation matrix A at step 646 is then sent back to step 636 in order to provide a circuitous or continuous cycle for continuously estimating and updating the correlation matrix A. The process in FIG. 7B is iterative, such that the correlation matrix A is constantly updating and becoming more accurate through each iteration. The correlation matrix A is generally updated based on the calculated intermediate catheter motion, and any one or more of the following steps: estimating apnea segments based on the RV; estimating stability weights based on the intermediate catheter motion; and regressing the correlation matrix A based on A = (FCM-ICM)/RV.

In one aspect, it is assumed that the 3D RCV can be modeled as a product of the 2D RV (which equals the main respiration signal + a phase shift) and the correlation matrix A (2x3). The parameters of the correlation matrix A are estimated based on the RV, a rough estimation of the respiration vector (which equals the filtered catheter motion minus the intermediate catheter motion), and the stability weights vector by minimizing the mean square error. By providing inputs of the (i) FCM, (ii) intermediate catheter motion, and (iii) stability weights, the correlation matrix A is generated as an output.

The intermediate catheter motion assumes relatively slow accelerations of the catheter, and cannot represent the true catheter motion in segments of high speed. An intermediate catheter motion error factor can be estimated based on segments of faster motion. Catheter motion is recalculated while increasing the curvature for high speed segments to allow for fast motion as well. Using this approach, the ICM error (ICMerr) = Norm(FCM - ICM - RCV). In other words, the respiration data equals total catheter motion minus intermediate catheter motion, and total catheter motion minus respiration equals in-heart catheter motion. In one embodiment, ICMerr is the per sample Euclidian distance in R3 between the measured location FCM and the slow model reconstructed location ICM + RCV. SmoothV is used to correct the intermediate catheter motion to find the in-heart catheter motion, in one aspect.

Station finding, also known as location finding or site finding, generally includes identifying periods when the catheter or probe is stable. This process includes performing logic and functions that produce stations' time edges and center locations based on the compensated motion. The stations in the last session are continuously updated along with the correlation matrix A and the resulting compensated motion. Stability is generally defined as a continuous segment of time in which the estimated catheter speed is below a user predefined threshold.

The catheter speed is calculated by taking a derivative of the catheter position over the last minute. As one example, the last-minute locations are updated at least every 0.1 second, and are saved for the whole minute. These values can vary depending on the specific requirements of a particular application. The last catheter speed value from this buffer is saved and accumulated in the last speed buffer.

For example, in one embodiment, the end-5 value in the catheter speed buffer is the speed of the catheter using the end-5 and end-7 values in the catheter one-minute locations buffer that were updated in current iteration. Here, the end-5 value in the last speed buffer is the last catheter speed that was calculated 5 iterations ago.

A minimum value of the catheter speed and the last speed over the last minute is defined as the minimal speed, and is used to detect when the catheter is stable. A minimal speed buffer includes all the minimum speeds that were estimated during the last minute. Segments of continuous speed that are smaller than a threshold speed are identified in this buffer, while trying to find a time period when the catheter was stable.

An estimation close to the current time is not accurate enough because speed can change dramatically near the buffer end without a reliable indication on the estimated speed. Accordingly, any determination regarding stability can be delayed for at least one second, and this predetermined delay can vary.

Boundaries of a current stable segment can be updated and then terminated if necessary. If a current stable segment was not found or if it was previously terminated, then a search for new stability segment in the recent several seconds of the estimated catheter motion is carried out.

If a stability segment was already found, then the boundaries are updated according to an updated minimal speed buffer. The starting point of the current stable segment can also be updated by looking backwards from the last index of stable segments until the earliest point when the speed is lower than a threshold.

The ending point of the stability segment can be updated by looking forward from the last index of a stable end until the latest point that the catheter speed is lower than a threshold.

A stable segment is not terminated until a predetermined period (i.e., roughly one second) occurs from the current time in order to avoid terminating segments due to edge inaccuracy. If a stable segment was not yet found, then the search for stability from the current time backward is carried out to find the earliest moment when a predetermined threshold was crossed.

In addition to the above process for determining when a segment is stable, the present disclosure also is directed to defining a localized segment as a time segment in which all its points (i.e., distance from its own center of gravity) are below a threshold. In other words, when considering a curved line in 3D space, the XYZ mean of the curved line can be calculated and this is the center of gravity. From this value, it is possible to calculate the distance from center (DFC) for every point on the line.

This process involves locating a site (i.e., a time segment) when the ablator is on and the catheter is both stable and localized. One aspect of this process is described in more detail herein. In one aspect, a stable time segment during ablation is identified.

The processes and algorithms disclosed herein can divide, segment, or otherwise split the stability segment during ablation into localized segments (i.e., sites) for which the maximal DFC is below a predetermined threshold. In one aspect, the predetermined threshold is 3.0 mm. One skilled in the art would understand that this value can vary. The algorithm for splitting the stability segment into sites essentially obtains the minimal number of sub-segments that maintain a localization criterion. In one aspect, it is a recursive function that ceases or stops in the event that the current sub-segment is localized. In one embodiment, this involves determining whether the maximum DFC is less than a threshold, i.e., 3.0 mm in one aspect.

In one aspect, an algorithm is provided that essentially splits or segments a curve in 3D dimensional space (i.e., the in-heart motion 50, catheter-heart motion, probe-cavity motion, etc.) into localized curves. As used herein, the term curve is defined as a vector of XYZ coordinates in 3D space that represents the course of motion of an object. In one aspect, this process involves determining the center of gravity of a curve defined by a data set, in which the center of gravity is defined as a point in 3D space that represents the mean value of each coordinate of the curve. The DFC is defined as a one-dimensional vector that represents the Euclidean distance between each point in the curve relative to the center of gravity, in one aspect. Finally, a localized curve is defined as a curve for which a maximum DFC is smaller than a predetermined threshold distance. In one aspect, this distance is 3.0 mm.

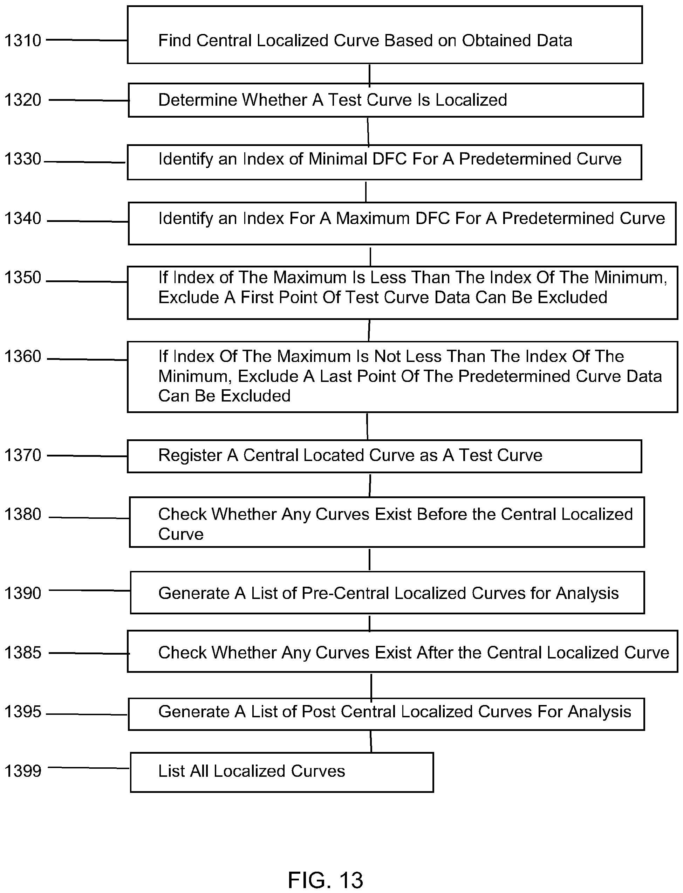

In one aspect, defining a split curve of 3D dimensional data is a recursive function. This defining a split curve is provided in the flow diagram 1300 of FIG. 13. First, the flow diagram 1300 includes finding a central localized curve based on the obtained data at 1310 (i.e., in-heart motion 50, catheter-heart motion, probe-cavity motion, etc.). Step 1310 can include determining whether a test curve (i.e., input curve) defined by the data set is localized at 1320, i.e., whether a maximum DFC is smaller than a threshold. During 1310, an index of a minimal DFC for a predetermined curve can be identified at 1330 and an index for a maximum DFC for a predetermined curve can be identified at 1340. If the index of the maximum is less than the index of the minimum (i.e., if the maximal DFC is before the minimal DFC), then a first point of the test curve data can be excluded at 1350, i.e., data points are set aside. If the index of the maximum is not less than the index of the minimum, then a last point of the predetermined curve data can be excluded at 1360, i.e., set aside. Flow diagram 1300 recognizes or registers a central localized curve as the test curve at 1370. The test curve, which is basically a potential candidate for the central localized curve, is iteratively cut from either side until it is localized and therefore declared or identified as the central localized curve. In certain situations, an input curve or the initial test curve will be the localized immediately, and therefore the cutting step can be omitted. In these situations, the input curve is the central localized curve, and therefore the splitting algorithm is not required.

Flow diagram 1300 checks whether any curves exist before the central localized curve at 1380. In one aspect, this step is performed based on the excluded or set aside pre-central points (disclosed above with respect to an index comparison step) by using a splitting function in a recursive manner. The process generates a list of pre-central localized curves for analysis at 1390 based on 1380. Flow diagram 1300 checks whether any curves exist after the central localized curve at 1385. In one aspect, this step is performed based on the excluded or set aside post-central points by using a splitting function in a recursive manner. The process generates a list of post-central localized curves for analysis at 1395 based on 1385. The list of all localized curves includes preceding localized curves, central localized curves, and proceeding localized curves at 1399. In other words, the algorithm essentially segments or breaks a 3D curve into a number of localized curves for further analysis. In particular, the analysis determines whether curves have a maximal DFC that is less than a predetermined distance threshold.

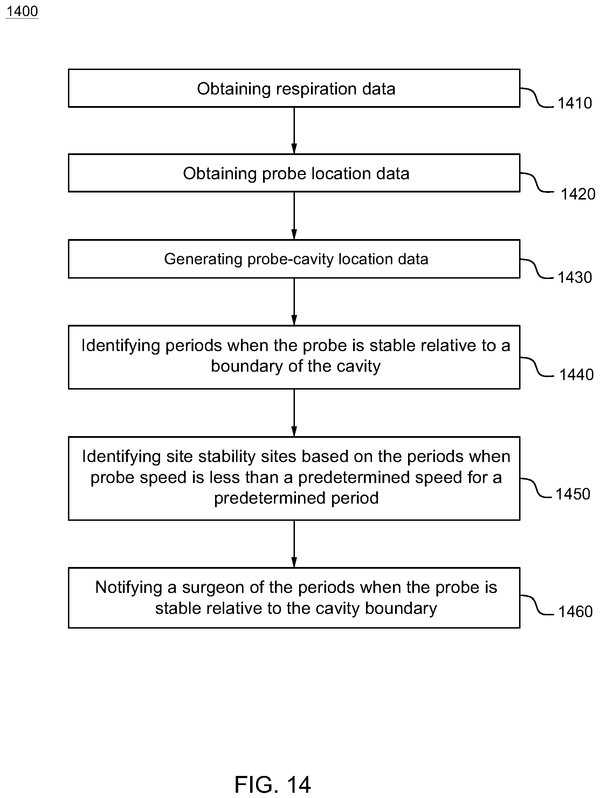

FIG. 14 illustrates a flow diagram illustrates various steps for a method 1400 for accounting for or minimizing the impact of the respiration component in the catheter motion data. As shown in FIG. 14, step 1410 includes obtaining respiration data. This data can be obtained by sensors attached to a patient's body. In one aspect, this data is obtained by a series of patches or sensors (i.e., sensors 18 from FIG. 1) attached to a patient's body that are configured to detect impedance values based on the patient's lungs filling with air. In another aspect, this data is obtained via a sensor arrangement including a catheter integrated with a sensor as well as external sensors, or solely based on a sensor integrated with a sensor. Breathing is generally based on a patient's diaphragm moving up and down, which also displaces the patient's heart. Accordingly, obtaining the respiration data is important for mapping the motion of a catheter within a patient's heart during respiration.

Step 1420 includes obtaining probe location data, which can be generated via any known tracking method or system. The probe location data can also be obtained using sensors (i.e., sensors 18 from FIG. 1) attached to a patient's body. In one aspect, the sensors are configured to generate a magnetic field and motion of the catheter inside of patient can generate magnetic signals to indicate a position of the catheter.

Step 1430 includes generating probe-cavity location data by compensating (e.g., subtracting or adding) the respiration data from the probe location data. Step 1440 includes identifying periods when the probe is stable relative to a boundary of the cavity. Step 1450 includes identifying site stability sites based on the periods when probe speed is less than a predetermined speed for a predetermined period. In one aspect, the predetermined speed can be 2 mm/second and the predetermined period can be at least three seconds. In one embodiment, the method 1400 includes applying a filter to the respiration data and the probe location data.

Step 1460 includes notifying a surgeon of the periods when the probe is stable relative to the cavity boundary. This step can include providing notifications via visual alerts or indicia (which can be displayed via monitor 3), audio alerts or indicia (which can be played via computing system 4), or any other notification such that the surgeon is informed of the stable periods. In one aspect, markers, tags, or visual indicators are overlaid or inserted onto a three-dimensional mapping or image of the location data and are displayed on a monitor (e.g., monitor 3 in FIG. 1).

Step 1410, described above, can further include converting respiration indicators by using singular value decomposition (SVD) to obtain a first Eigenvector and a first Eigenvector derivative. As would be understood by those possessing an ordinary skill in the art, the first Eigenvector can correspond to a primary respiration signal and the first Eigenvector derivative can correspond to a phase shifted respiration signal. The method 1400 can include transferring the primary respiration signal and the phase shifted respiration signal from two dimensions into a three-dimensional ellipsoid based on a correlation matrix. The method 1400 can also include determining the correlation matrix by calculating a root mean square deviation between the probe location data and the probe-cavity motion data. The method 1400 can include determining the correlation matrix based on weighted factors including at least one of: (i) age of the respiration data; (ii) speed of the probe; (iii) depth of respiration; or (iv) data obtained during ablation. In one embodiment, the method 1400 includes generating an image including estimated respiratory motion based on the respiration data, probe motion based on the probe location data, and probe-cavity motion based on the probe-cavity location data. This image can then be displayed on a monitor to a surgeon, along with indicators, tags, or visual indicators showing periods when the probe is stable relative to the cavity boundary.

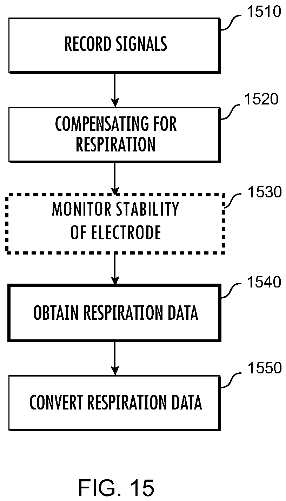

FIG. 15 illustrates a method 1500 of capturing mapping data with respiration compensation. In a mapping configuration, as described above, the predetermined speed of the probe location data may be small, and even may be zero. Further, in a mapping configuration, where ablation is absent, the input for the ablation is also zero. Therefore, in such a configuration, the movement of the electrodes in relation to the tissue wall may largely be a result of respiratory motion.

At step 1510, in a mapping configuration, the signal(s) from multiple electrodes are recorded over a period of about 2.5 seconds. As a result of the use of 2.5 seconds periods, the beating of the heart may be accounted for. As a result of the duration accounting for the beating of the heart, the movement due to respiration is the main remaining factor effecting the mapping. This movement due to respiration can cause a smearing of the data.

At step 1520, in order to reduce, or eliminate, the smearing of the data, the respiration movement can be compensated for. As described above in FIG. 2, this motion can be significant. Since the respiration motion is known and understood, as described above, the mapping points collected during the respiration motion may be compensated for. For example all of the points captured during the respiration cycle may be adjusted to the end of respiration phase to produce a better anatomical mapping (less blurry). In such a configuration, by adjusting the data points to remove the respiration movement, the picture clarity is improved. As will be understood, the compensation of the mapping points could be positioned at a reference point during any portion of the respiration cycle, including the beginning, the midpoint, or any other portion of the respiration cycle. The end of the respiration is used as an example to aid in the understanding of the description. This data collection may be referred to as compensated motion to mapping.

At step 1530, additionally, or alternatively, the stability of each electrode (or spline of a catheter) may be monitored as described herein. When data is collected, the stability is used to determine the mapping values. This determination may include at least one of adjusting the mapped data points based on the stability and only computing mapping values for electrodes that are deemed to be stable. For example, when a data collection occurs, the data from stable electrodes may be recorded, while the less than stable electrodes are excluded, or have their data recollected. Alternatively, a weighting system may be used based on the level of stability in collecting the data. In either example - the wait or the weight, which may also be employed in unison, the data is improved and less blurry. Using a gating function based on the stability the data collected is of improved quality.

At step 1540 respiration data may be obtained. In order to compensate for respiration data at step 1550, the respiration data may be obtained. This data can be obtained by sensors attached to a patient's body. In one aspect, this data is obtained by a series of patches or sensors (i.e., sensors 5 from FIG. 1) attached to a patient's body that are configured to detect impedance values based on the patient's lungs filling with air. In another aspect, this data is obtained via a sensor arrangement including a catheter integrated with a sensor as well as external sensors, or solely based on a sensor integrated with a sensor. Breathing is generally based on a patient's diaphragm moving up and down, which also displaces the patient's heart. Accordingly, obtaining the respiration data is important for mapping the motion of a catheter within a patient's heart during respiration.

At step 1550 respiration data may be converted to indicators by using singular value decomposition (SVD) to obtain a first Eigenvector and a first Eigenvector derivative. The first Eigenvector can correspond to a primary respiration signal and the first Eigenvector derivative can correspond to a phase shifted respiration signal. The method 1400 can include transferring the primary respiration signal and the phase shifted respiration signal from two dimensions into a three-dimensional ellipsoid based on a correlation matrix. The method 1400 can also include determining the correlation matrix by calculating a root mean square deviation between the probe location data and the probe-cavity motion data. The method 1400 can include determining the correlation matrix based on weighted factors including at least one of: (i) age of the respiration data; (ii) speed of the probe; (iii) depth of respiration; or (iv) data obtained during ablation. In one embodiment, the method 1400 includes generating an image including estimated respiratory motion based on the respiration data, probe motion based on the probe location data, and probe-cavity motion based on the probe-cavity location data. This image can then be displayed on a monitor to a surgeon, along with indicators, tags, or visual indicators showing periods when the probe is stable relative to the cavity boundary.

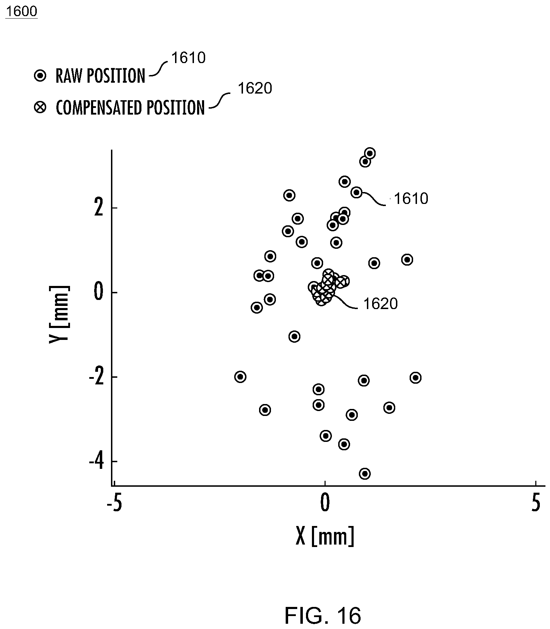

FIG. 16 illustrates a plot 1600 of respiration data points with and without respiration compensation. Plot 1600 includes data in the X-axis from -5 to +5 mm. Plot 1600 includes data in the Y-axis from -4 to +4 mm. In plot 1600 data points 1610 represent data with no respiration compensation. Data points 1620 represent the same data with compensation. Additionally, the data points 1620 include points where the data is shifted from the continuum of the respiration cycle to a point say at the end of the cycle. As illustrated, the data points 1610 without respiration compensation have a Y-axis spread of approximately 8 mm and an X-axis spread of approximately 5 mm. Data points 1620 with the compensation have a Y-axis spread of less than 0.5 mm and an X-axis spread of less than 0.5 mm.

The disclosed subject matter is not limited to being used in connection with a heart. The disclosed subject matter can be used in a variety of applications to analyze features of any type of object, such as a chamber.

Any of the functions and methods described herein can be implemented in a general-purpose computer, a processor, or a processor core. Suitable processors include, by way of example, a general-purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums include a read only memory (ROM), a random-access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A method comprising:

   obtaining respiration data of a patient via at least one sensor;
   obtaining probe location data for a probe positioned within a cavity;
   generating compensated location data by compensating the obtained probe location data with the obtained respiration data; and
   producing a mapping based on the generated compensated location data.

2. The method according to claim 1, further comprising applying a filter to the respiration data.

3. The method according to claim 1, wherein the obtaining respiration data further includes: obtaining respiration indicators via the at least one sensor.

4. The method according to claim 3, wherein the obtaining respiration data further includes: converting the respiration indicators by using singular value decomposition (SVD) to obtain a first Eigenvector and a first Eigenvector derivative, the first Eigenvector corresponding to a primary respiration signal and the first Eigenvector derivative corresponding to a phase shifted respiration signal, optionally wherein the primary respiration signal and the phase shifted respiration signal are transferred from two dimensions into a three-dimensional ellipsoid based on a correlation matrix, further optionally wherein the correlation matrix is determined based on weighted factors including at least one of: age of the respiration data; speed of the probe; and depth of respiration.

5. The method according to claim 1, further comprising generating an image including:

   estimated respiratory motion based on the respiration data,
   probe motion based on the probe location data, and
   probe-cavity motion based on the probe-cavity location data.

6. The method according to claim 1, further comprising generating a notification when the probe is stable relative to a boundary of the cavity, optionally wherein notifying periods when the probe is stable relative to the boundary via visual indicators displayed on a monitor.

7. The method according to claim 1, further comprising identifying site stability sites based on periods when probe speed is less than a predetermined velocity for at least a predetermined period of time.

8. A system comprising:

   a probe configured to be inserted into an intra-body cavity of a patient;
   at least one sensor configured to obtain respiration data, the probe and the at least one sensor being configured to obtain probe location data; and

a processor configured to:

generate compensated location data by compensating the obtained probe location data with the obtained respiration data, and
produce a mapping based on the generated compensated location data.

9. The system according to claim 8, wherein the processor is further configured to apply a filter to the respiration data and the probe location data.

10. The system according to claim 8, wherein the processor is further configured to generate respiration indicators based on the respiration data from the at least one sensor.

11. The system according to claim 10, wherein the processor is further configured to convert the respiration indicators by using singular value decomposition (SVD) to obtain a first Eigenvector and a first Eigenvector derivative, the first Eigenvector corresponding to a primary respiration signal and the first Eigenvector derivative corresponding to a phase shifted respiration signal.

12. The system according to claim 11, wherein the primary respiration signal and the phase shifted respiration signal are transferred from two dimensions into a three-dimensional ellipsoid based on a correlation matrix, optionally wherein the correlation matrix is determined based on weighted factors including at least one of: age of the respiration data; speed of the probe; and depth of respiration.

13. The system according to claim 8, wherein the processor is further configured to generate an image including estimated respiratory motion based on the respiration data, probe motion based on the probe location data, and probe-cavity motion based on the probe-cavity location data.

14. The system according to claim 8, wherein the processor is further configured to notify of the periods when the probe is stable relative to a boundary of the cavity, optionally wherein notifying of the periods when the probe is stable relative to the boundary includes displaying visual indicators on a monitor.

15. The system according to claim 8, wherein the processor is further configured to identify site stability sites based on the periods when probe speed is less than 2 mm/second for at least three seconds.

FIG. 1

FIG. 2

EP 4 566 533 A1

200

FIG. 3

EP 4 566 533 A1

300

310

312

314

318

316

320

322

324

326

350

FIG. 4A

FIG. 4B

FIG. 5

FIG. 6

600

FIG. 7A

FIG. 7B

Respiration Vectors - apnea during ablation

FIG. 8

Recency Factor

FIG. 9

FIG. 10

FIG. 11

Stability Weights Factors

FIG. 12

EP 4 566 533 A1

1310 — Find Central Localized Curve Based on Obtained Data

1320 — Determine Whether A Test Curve Is Localized

1330 — Identify an Index of Minimal DFC For A Predetermined Curve

1340 — Identify an Index For A Maximum DFC For A Predetermined Curve

1350 — If Index of The Maximum Is Less Than The Index Of The Minimum, Exclude A First Point Of Test Curve Data Can Be Excluded

1360 — If Index Of The Maximum Is Not Less Than The Index Of The Minimum, Exclude A Last Point Of The Predetermined Curve Data Can Be Excluded

1370 — Register A Central Located Curve as A Test Curve

1380 — Check Whether Any Curves Exist Before the Central Localized Curve

1390 — Generate A List of Pre-Central Localized Curves for Analysis

1385 — Check Whether Any Curves Exist After the Central Localized Curve

1395 — Generate A List of Post Central Localized Curves For Analysis

1399 — List All Localized Curves

FIG. 13

1400

```
┌─────────────────────────────────────────────────┐
│          Obtaining respiration data              │──── 1410
└─────────────────────────────────────────────────┘
                        │
                        ▼
┌─────────────────────────────────────────────────┐
│          Obtaining probe location data           │──── 1420
└─────────────────────────────────────────────────┘
                        │
                        ▼
┌─────────────────────────────────────────────────┐
│        Generating probe-cavity location data     │──── 1430
└─────────────────────────────────────────────────┘
                        │
                        ▼
┌─────────────────────────────────────────────────┐
│  Identifying periods when the probe is stable    │
│  relative to a boundary of the cavity            │──── 1440
└─────────────────────────────────────────────────┘
                        │
                        ▼
┌─────────────────────────────────────────────────┐
│  Identifying site stability sites based on the   │
│  periods when probe speed is less than a         │
│  predetermined speed for a predetermined period  │──── 1450
└─────────────────────────────────────────────────┘
                        │
                        ▼
┌─────────────────────────────────────────────────┐
│  Notifying a surgeon of the periods when the     │
│  probe is stable relative to the cavity boundary │──── 1460
└─────────────────────────────────────────────────┘
```

FIG. 14

FIG. 15

1600

⊙ RAW POSITION ⟋ 1610

⊗ COMPENSATED POSITION ⟋ 1620

FIG. 16

Europäisches
Patentamt

European
Patent Office

Office européen
des brevets

# EUROPEAN SEARCH REPORT

**Application Number**

EP 24 21 5980

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/183669 A1 (BAR-TAL MEIR [IL] ET AL) 16 June 2022 (2022-06-16) * paragraphs [0006], [0033] - [0037], [0042] - [0047], [0054], [0066] - [0067], [0079] - [0080], [0133] - [0136] * | 1-15 | INV. A61B5/113 A61B5/287 A61B5/367 A61B5/00 |
| X | US 2013/296845 A1 (BAR-TAL MEIR [IL] ET AL) 7 November 2013 (2013-11-07) * paragraphs [0026], [0031], [0037], [0043] * | 1-15 | |
| A | US 2012/197111 A1 (BAR-TAL MEIR [IL]) 2 August 2012 (2012-08-02) * the whole document * | 1-15 | |
| X | US 2022/125523 A1 (NAKAR ELAD [IL] ET AL) 28 April 2022 (2022-04-28) * paragraphs [0076], [0097], [0103] * | 1-15 | |
| X | US 2004/254437 A1 (HAUCK JOHN A [US] ET AL) 16 December 2004 (2004-12-16) * paragraphs [0014], [0020], [0039], [0051], [0073] * | 1-15 | **TECHNICAL FIELDS SEARCHED (IPC)** A61B |

The present search report has been drawn up for all claims

| Place of search | Date of completion of the search | Examiner |
|---|---|---|
| Berlin | 16 April 2025 | Clevorn, Jens |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 21 5980

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2025

| Patent document cited in search report | Publication date | Patent family member(s) | | Publication date |
|---|---|---|---|---|
| US 2022183669 A1 | 16-06-2022 | CN | 114631802 A | 17-06-2022 |
| | | EP | 4014857 A1 | 22-06-2022 |
| | | IL | 288702 A | 01-07-2022 |
| | | JP | 2022095582 A | 28-06-2022 |
| | | US | 2022183669 A1 | 16-06-2022 |
| US 2013296845 A1 | 07-11-2013 | AU | 2013205728 A1 | 21-11-2013 |
| | | CA | 2815755 A1 | 07-11-2013 |
| | | CN | 103385705 A | 13-11-2013 |
| | | EP | 2662049 A2 | 13-11-2013 |
| | | JP | 6328375 B2 | 23-05-2018 |
| | | JP | 6517382 B2 | 22-05-2019 |
| | | JP | 2013233436 A | 21-11-2013 |
| | | JP | 2018061874 A | 19-04-2018 |
| | | US | 2013296845 A1 | 07-11-2013 |
| US 2012197111 A1 | 02-08-2012 | NONE | | |
| US 2022125523 A1 | 28-04-2022 | CN | 114376579 A | 22-04-2022 |
| | | EP | 3988025 A1 | 27-04-2022 |
| | | IL | 287360 A | 01-05-2022 |
| | | JP | 2022068863 A | 10-05-2022 |
| | | US | 2022125523 A1 | 28-04-2022 |
| US 2004254437 A1 | 16-12-2004 | NONE | | |

EPO FORM P0459

For more details about this annex : see Official Journal of the European Patent Office, No. 12/82

**EP 4 566 533 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B
- US 5391199 A
- US 6177792 B
- US 5944022 A
- US 5983126 A
- US 6456864 B